(19) Europäisches Patentamt
European Patent Office
Office européen des brevets

(11) EP 3 957 618 A1

(12) EUROPÄISCHE PATENTANMELDUNG

(43) Veröffentlichungstag:
23.02.2022 Patentblatt 2022/08

(21) Anmeldenummer: 21191484.1

(22) Anmeldetag: 16.08.2021

(51) Internationale Patentklassifikation (IPC):
C04B 35/115 (2006.01)   A61C 13/083 (2006.01)
A61K 6/807 (2020.01)   A61K 6/822 (2020.01)

(52) Gemeinsame Patentklassifikation (CPC):
C04B 35/115; A61C 13/0022; A61C 13/083;
A61K 6/807; A61K 6/822; C04B 2235/3206;
C04B 2235/3224; C04B 2235/3227;
C04B 2235/3229; C04B 2235/3241;
C04B 2235/3244; C04B 2235/3262;
C04B 2235/3272; C04B 2235/3275;
C04B 2235/3281;                    (Forts.)

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME
Benannte Validierungsstaaten:
KH MA MD TN

(30) Priorität: 21.08.2020 DE 102020121993

(71) Anmelder: Ivoclar Vivadent AG
9494 Schaan (LI)

(72) Erfinder:
• Reinshagen, Jörg
  75177 Pforzheim (DE)
• Niebergall, Rick
  74372 Sersheim (DE)

(74) Vertreter: Uexküll & Stolberg
Partnerschaft von
Patent- und Rechtsanwälten mbB
Beselerstraße 4
22607 Hamburg (DE)

(54) ALUMINIUMOXID-KERAMIKMATERIAL

(57) Die Erfindung betrifft ein Aluminiumoxid-Keramikmaterial, das die folgenden Komponenten enthält:

| Komponente | Gew.-% |
|---|---|
| $Al_2O_3$ | 95,0 bis 99,989 |
| $MgO$ | 0,001 bis 0,1 |
| Eu, berechnet als $Eu_2O_3$ | 0,01 bis 1,0. |

Fig. 1

(52) Gemeinsame Patentklassifikation (CPC): (Forts.)
    C04B 2235/3291; C04B 2235/606; C04B 2235/616; C04B 2235/6562; C04B 2235/6565; C04B 2235/75;
    C04B 2235/786; C04B 2235/96; C04B 2235/9661

**Beschreibung**

Die vorliegende Erfindung betrifft ein Aluminiumoxid-Keramikmaterial, das innerhalb sehr kurzer Zeit dichtgesintert werden kann und sich aufgrund der dabei erhaltenen ausgezeichneten optischen Eigenschaften insbesondere zur Herstellung von dentalen Restaurationen eignet, sowie ein Verfahren zu dessen Herstellung. Die Erfindung betrifft auch ein Verfahren zur Herstellung eines gesinterten Aluminiumoxid-Keramikkörpers unter Verwendung des erfindungsgemäßen Keramikmaterials sowie die Verwendung dieses Keramikmaterials zur Herstellung einer dentalen Restauration.

Zur Herstellung vollanatomischer Dentalrestaurationen werden häufig keramische Materialien wie Aluminiumoxid-Keramiken eingesetzt. Diese bieten eine hohe klinische Sicherheit, sind üblicherweise metallfrei, können auch bei minimal-invasiven Präparationen eingesetzt werden und sind preislich im Vergleich zu anderen metallfreien Restaurationen sehr attraktiv. Nachteilig sind jedoch die langwierigen Arbeitsschritte, die meist zur Herstellung solcher Restaurationen erforderlich sind.

Üblicherweise werden die Restaurationen aus vorgesinterten Rohlingen herausgefräst oder geschliffen, gegebenenfalls eingefärbt, durch thermische Behandlung dichtgesintert und schließlich gegebenenfalls weiter eingefärbt, glasiert und/oder poliert.

Um die für einen industriellen oder medizinischen Einsatz erforderlichen mechanischen Eigenschaften einer Aluminiumoxidkeramik bereitzustellen, wird häufig eine heißisostatische Nachverdichtung des zuvor gesinterten Materials durchgeführt. Dabei wird das bis zum Porenabschluss gesinterte feinkörnige Aluminiumoxid typischerweise bei 1200°C bis 1300°C und sehr hohen Drücken von 1000 bis 2000 bar unter Schutzgas, in der Regel Argon oder Stickstoff, nachverdichtet. Ein entsprechendes Verfahren ist beispielsweise in WO 2007/065914 A1 beschrieben. Eine solche heißisostatische Nachverdichtung erfordert jedoch einen großen technischen Aufwand und eignet sich daher nicht für eine Chairside-Behandlung direkt beim Zahnarzt.

Alternativ kann Aluminiumoxid auch unter Wasserstoffatmosphäre bei sehr hohen Temperaturen, typischerweise zwischen 1700°C bis 1900°C, transparent gesintert werden. Allerdings zeigen die so erhaltenen Keramiken in der Regel relativ geringe Festigkeiten. Zudem erfordern auch diese Verfahren einen sehr hohen technischen Aufwand und sind daher ebenfalls nicht für eine Chairside-Behandlung direkt beim Zahnarzt geeignet.

Konventionelle Sinterverfahren für Dentalkeramiken beinhalten ein langsames Aufheizen auf eine Maximaltemperatur, bei der das eingesetzte Oxidkeramikmaterial dichtgesintert wird. Bedingt durch die geringe Aufheizgeschwindigkeit dauert ein solcher Sinterprozess typischerweise deutlich mehr als 4 Stunden und trägt dadurch erheblich zu einer vor allem bei Chairside-Behandlungen unbefriedigend großen Dauer des Produktionszyklus von Dentalkeramiken bei.

Ansätze zur Beschleunigung des Sinterprozesses durch Erhöhung der Aufheizgeschwindigkeit sind grundsätzlich bekannt.

So beschreibt EP 2 098 188 A1 einen Dentalofen und ein Verfahren zum Sintern von Dentalmaterialien, bei dem der Ofen in einer ersten Aufheizperiode mit einer schnellen Aufheizrate von mehr als 50 K/min auf eine Vorsintertemperatur von mindestens 1000°C aufgeheizt wird.

EP 2 101 133 A1 beschreibt einen Sinterofen und ein Verfahren zum Sintern von Dentalpräparaten, bei dem die Dentalpräparate entlang einer Sinterstrecke bewegt und dabei verschiedenen Temperaturen ausgesetzt werden. Dabei können in einem ersten Abschnitt hohe Aufheizraten von 300 K/min oder mehr eingesetzt werden.

WO 2012/057829 A2 beschreibt ein Verfahren zum schnellen Sintern von Keramik unter Verwendung von elektromagnetischer Induktion oder eines Plasmas.

WO 2015/091744 A1 beschreibt ein Verfahren zur Planung einer Sinterung eines Zahnersatzteils, bei dem in Abhängigkeit von bestimmten Geometrie- und Materialparametern des herzustellenden Zahnersatzteils ein Temperaturprofil für die Wärmebehandlung des Zahnersatzteils automatisch mittels eines Computers bestimmt wird. Dabei wird für die Sinterung bestimmter Zahnersatzteile eine Heizrate zwischen 100 K/min und 400 K/min verwendet.

WO 2015/121364 A1 beschreibt einen Sinterofen für Dentalbauteile mit einer Aufheizvorrichtung, die im Nutzbereich eine Aufheizrate von mindestens 200 K/min ermöglicht.

Allerdings führen die bekannten Ansätze zur Beschleunigung des Sinterprozesses typischerweise zu einer dunklen, grau-braunen Verfärbung und damit einhergehend zu einem Transluzenzverlust, so dass Keramikmaterialien erhalten werden, deren Eigenschaften vor allem in optischer Hinsicht den hohen Anforderungen insbesondere im Dentalbereich nicht genügen.

Es ist versucht worden, der beschriebenen Verfärbung durch eine auf den Sinterprozess folgende zusätzliche Wärmebehandlung entgegenzuwirken. Allerdings führt dies zu einer insbesondere für Chairside-Behandlungen inakzeptablen Verlängerung des Produktionszyklus.

Der Erfindung liegt daher die Aufgabe zugrunde, ein Aluminiumoxid-Keramikmaterial bereitzustellen, das innerhalb sehr kurzer Zeit vorzugsweise unter Umgebungsdruck und Umgebungsatmosphäre ohne heißisostatische Nachverdichtung mit ausgezeichneten optischen Eigenschaften dichtgesintert werden kann und insbesondere die Herstellung von dentalen Restaurationen mit hervorragenden mechanischen und insbesondere optischen Eigenschaften im Rahmen einer Chairside-Behandlung ermöglicht.

Diese Aufgabe wird erfindungsgemäß gelöst durch das Aluminiumoxid-Keramikmaterial nach den Ansprüchen 1 bis 12. Gegenstand der Erfindung ist auch das Verfahren zur Herstellung des erfindungsgemäßen Aluminiumoxid-Keramikmaterials nach Anspruch 13 oder 14, das Verfahren zur Herstellung eines gesinterten Aluminiumoxid-Keramikkörpers nach den Ansprüchen 15 bis 18 sowie die Verwendung des erfindungsgemäßen Aluminiumoxid-Keramikmaterials zur Herstellung einer dentalen Restauration nach Anspruch 19.

Das erfindungsgemäße Aluminiumoxid-Keramikmaterial zeichnet sich dadurch aus, dass es die folgenden Komponenten enthält:

| Komponente | Gew.-% |
|---|---|
| $Al_2O_3$ | 95,0 bis 99,989 |
| MgO | 0,001 bis 0,1 |
| Eu, berechnet als $Eu_2O_3$ | 0,01 bis 1,0. |

Es wurde überraschend gefunden, dass das erfindungsgemäße Aluminiumoxid-Keramikmaterial mit Gehalt an Europium eine sehr schnelle Sinterung zu gesinterten Aluminiumoxid-Keramikkörpern ermöglicht, die gute mechanische und optische Eigenschaften aufweisen. Vor allem kann das erfindungsgemäße Aluminiumoxid-Keramikmaterial mit Gehalt an Europium nach entsprechender Formgebung in einem nur sehr kurzen Zeitraum sogar unter Umgebungsdruck und Umgebungsatmosphäre und ohne die Notwendigkeit einer thermischen Nachbehandlung zu dentalen Restaurationen dichtgesintert werden, die dennoch sehr gute optische Eigenschaften und insbesondere eine hohe Transluzenz aufweisen und damit auch in ästhetischer Hinsicht die hohen Anforderungen an dentale Restaurationen erfüllen. Damit ist es möglich, für einen Patienten innerhalb nur einer Sitzung beim Zahnarzt eine sowohl gewünscht geformte als auch dichtgesinterte Dentalrestauration aus einem Rohling zu erzeugen und diese bei ihm einzusetzen. Eine derartig schnelle Versorgung mit einer Dentalrestauration wird auch als Chairside-Behandlung bezeichnet und ist für den Patienten naturgemäß überaus attraktiv. Damit ist das erfindungsgemäße Aluminiumoxid-Keramikmaterial mit Gehalt an Europium konventionellen Aluminiumoxid-Keramikmaterialien deutlich überlegen, bei denen gerade zur Erzielung einer zufriedenstellenden Transluzenz sehr lange Sinterzeiten in Kauf genommen werden müssen.

Es ist bevorzugt, dass das erfindungsgemäße Aluminiumoxid-Keramikmaterial mindestens 96,4, insbesondere mindestens 97,4 und bevorzugt mindestens 98,4 Gew.-% $Al_2O_3$ enthält. Besonders bevorzugt ist ein Aluminiumoxid-Keramikmaterial, das 96,4 bis 99,9, insbesondere 97,4 bis 99,85, bevorzugt 98,4 bis 99,8, weiter bevorzugt 99,4 bis 99,75, besonders bevorzugt 99,5 bis 99,7 und am meisten bevorzugt 99,55 bis 99,65 Gew.-% $Al_2O_3$ enthält.

Weiter ist ein Aluminiumoxid-Keramikmaterial bevorzugt, das 0,005 bis 0,09, insbesondere 0,01 bis 0,08, bevorzugt 0,03 bis 0,07 und besonders bevorzugt 0,04 bis 0,06 Gew.-% MgO enthält.

Auch ist ein Aluminiumoxid-Keramikmaterial bevorzugt, das 0,05 bis 0,9, insbesondere 0,1 bis 0,8, bevorzugt 0,2 bis 0,7 und besonders bevorzugt 0,3 bis 0,6 Gew.-% Eu, berechnet als $Eu_2O_3$, enthält.

Weiter ist auch ein Aluminiumoxid-Keramikmaterial bevorzugt, das 0,01 bis 1,0, insbesondere 0,05 bis 0,5, bevorzugt 0,1 bis 0,4 und besonders bevorzugt 0,2 bis 0,3 Gew.-% Nd, berechnet als $Nd_2O_3$, enthält.

Das erfindungsgemäße Aluminiumoxid-Keramikmaterial enthält vorzugsweise 0,001 bis 2,0, insbesondere 0,01 bis 1,0, bevorzugt 0,05 bis 0,8 und besonders bevorzugt 0,1 bis 0,5 Gew.-% färbende Oxide, wobei die färbenden Oxide vorzugsweise ausgewählt sind aus Oxiden von Fe, Cr, Mn, Co, Cu, Ag, Er, Pr, Tb, Ce, Ho, Zr und La.

Auch ist es bevorzugt, dass das Aluminiumoxid-Keramikmaterial nicht mehr als 0,5, insbesondere nicht mehr als 0,3, bevorzugt nicht mehr als 0,1, besonders bevorzugt nicht mehr als 0,05 und am meisten bevorzugt nicht mehr als 0,01 Gew.-% andere Oxide enthält. Dabei bezeichnet der Begriff "andere Oxide" andere als die oben genannten Oxide, d.h. insbesondere Oxide mit Ausnahme von $Al_2O_3$, MgO und Oxiden von Eu, Nd, Fe, Cr, Mn, Co, Cu, Ag, Er, Pr, Tb, Ce, Ho, Zr und La.

Das erfindungsgemäße Aluminiumoxid-Keramikmaterial weist vorzugsweise eine zahlenmittlere Korngröße im Bereich von 0,1 bis 10,0 μm, insbesondere von 0,5 bis 5,0 μm und bevorzugt von 0,7 bis 2,0 μm auf. Die zahlenmittlere Korngröße kann insbesondere nach dem Linienschnittverfahren gemäß DIN EN 623-3 oder ASTM E 112 bestimmt werden, wobei der ermittelte Wert zur Umrechnung auf die reale zahlenmittlere Korngröße im dreidimensionalen Mikrogefüge nach M. I. Mendelson, J. Am. Ceram. Soc. 1969, 52(8), 443-446 mit einer Proportionalitätskonstante von 1,56 multipliziert wird.

In einer bevorzugten Ausführungsform enthält das erfindungsgemäße Aluminiumoxid-Keramikmaterial eine Europiumaluminat-Kristallphase, wobei der Begriff "Europiumaluminat" erfindungsgemäß auch Europium-Neodymaluminat umfasst. Diese Kristallphase enthält typischerweise stäbchenförmige oder plättchenförmige Europiumaluminat-Kristalle. Besonders bevorzugt enthält das Aluminiumoxid-Keramikmaterial stäbchenförmige Europiumaluminat-Kristalle, die bevorzugt eine mittlere Breite von 0,01 bis 0,25, insbesondere von 0,05 bis 0,20, bevorzugt 0,10 bis 0,15 und besonders bevorzugt 0,11 bis 0,13 μm haben und/oder ein mittleres Verhältnis von Breite zu Länge von 1:1 bis 1:10, insbesondere 1:2 bis 1:9, bevorzugt 1:4 bis 1:8 und besonders bevorzugt 1:6 bis 1:7 aufweisen.

Es ist weiter bevorzugt, dass das Aluminiumoxid-Keramikmaterial eingefärbt ist. Hierunter wird erfindungsgemäß ein Keramikmaterial verstanden, das mit einem oder mehreren färbenden Elementen versetzt ist. Beispiele für geeignete färbende Elemente sind Fe, Cr, Mn, Co, Cu, Ag, Er, Pr, Tb, Ce, Ho, Zr und La. Besonders bevorzugt umfasst das Aluminiumoxid-Keramikmaterial mindestens zwei, insbesondere mindestens drei und vorzugsweise mindestens vier Schichten, die sich in ihrer Farbe unterscheiden, und vorzugsweise einen Gradienten, der eine kontinuierliche Änderung der Farbe aufweist.

Im Sinne der vorliegenden Anmeldung beziehen sich die Begriffe "Farbe" und "eingefärbt" auf die Farbe, Helligkeit und/oder Transluzenz eines Materials.

"Transluzenz" ist die Lichtdurchlässigkeit eines Materials, Körpers oder einer Schicht, d.h. das Verhältnis von durchgelassener zu eingestrahlter Lichtintensität.

Farben können auch durch die Farbkoordinaten L*, a* und b* im L*a*b*-Farbraum oder durch einen in der Dentalindustrie üblicherweise verwendeten Farbcode charakterisiert werden. Im L*a*b*-Farbraum beschreibt der Wert L* die Helligkeit einer Farbe mit Werten von 0 (schwarz) bis 100 (weiß), der Wert a* den Grün- oder Rotanteil einer Farbe, wobei negative Werte für Grün und positive Werte für Rot stehen, und der Wert b* den Blau- oder Gelbanteil einer Farbe, wobei negative Werte für Blau und positive Werte für Gelb stehen. Beispiele für in der Dentalindustrie übliche Farbcodes sind Vitapan classical® und Vita 3D Master®, beide von der VITA Zahnfabrik H. Rauter GmbH & Co. KG, und Chromascop® von der Ivoclar Vivadent AG.

Üblicherweise erfolgen die Bestimmung der Farbkoordinaten L*, a* und b* nach DIN 5033 und DIN 6174 und die Bestimmung der Transluzenz nach BS 5612. Die entsprechenden Messungen können insbesondere mittels eines Spektrophotometers vom Typ CM-3700d (Konica-Minolta) durchgeführt werden. Hierzu werden für die Messungen Probenkörper eingesetzt, die mit Diamantpartikeln (Partikelgröße 15-20 μm) beidseitig nass beschliffen wurden, um eine finale Probendicke von 2,00 ± 0,025 mm oder vorzugsweise 1,00 ± 0,05 mm zu erhalten. Die Transluzenz berechnet sich aus dem nach BS 5612 bestimmten CR-Wert gemäß der Formel

$$\text{Transluzenz}[\%] = 100\% - \text{CR-Wert}[\%]$$

so dass eine Transluzenz von 0% vollständig opak und eine Transluzenz von 100% vollständig transluzent bedeutet.

Vorzugsweise liegen die Farbe oder die Farben der erfindungsgemäß erhaltenen dentalen Restauration im Bereich der Farben natürlicher Zähne. Besonders bevorzugt weisen der erfindungsgemäß erhaltene Aluminiumoxid-Keramikkörper und insbesondere die erfindungsgemäß erhaltene dentale Restauration einen L*-Wert im Bereich von 50 bis 100, insbesondere im Bereich von 80 bis 97, einen a*-Wert im Bereich von -10 bis 10, insbesondere im Bereich von -1 bis 5, einen b*-Wert im Bereich von 0 bis 50, insbesondere im Bereich von 1 bis 20, und/oder eine Transluzenz im Bereich von 35 bis 100%, insbesondere im Bereich von 40 bis 99%, bevorzugt im Bereich von 45 bis 98% und besonders bevorzugt im Bereich von 50 bis 97%, auf.

In einer weiteren Ausführungsform ist das Aluminiumoxid-Keramikmaterial ein nicht dichtgesintertes und insbesondere ein vorgesintertes Aluminiumoxid-Keramikmaterial. Üblicherweise weist das Aluminiumoxid-Keramikmaterial dabei eine relative Dichte im Bereich von 45 bis 90%, insbesondere im Bereich von 50 bis 80% und bevorzugt im Bereich von 55 bis 70%, jeweils bezogen auf die Reindichte des Aluminiumoxid-Keramikmaterials, auf.

Die relative Dichte ist dabei das Verhältnis der scheinbaren Dichte des Keramikmaterials zur Reindichte des Keramikmaterials.

Die scheinbare Dichte des Keramikmaterials kann mittels der Immersionsmethode gemäß ISO 18754 aus der Masse der trockenen Probe ($m_t$), der scheinbaren Masse der in einer Immersionsflüssigkeit untergetauchten Probe ($m_i$) und der Dichte der Immersionsflüssigkeit ($\rho_i$) nach der Formel

$$\rho = \frac{m_t}{m_t - m_i} \times \rho_i$$

berechnet werden. Als Immersionsflüssigkeit wird vorzugsweise Tetrachlorkohlenstoff ($CCl_4$) eingesetzt.

Die Bestimmung der Reindichte des Aluminiumoxid-Keramikmaterials erfolgt, indem das Aluminiumoxid-Keramikmaterial auf ein Pulver mit einer mittleren Teilchengröße von 10 bis 30 μm, insbesondere von 20 μm, bezogen auf die Anzahl der Teilchen, gemahlen und die Dichte des Pulvers mittels Pyknometer ermittelt wird.

Die Bestimmung der Teilchengröße kann beispielsweise mit dem CILAS® Particle Size Analyzer 1064 der Firma Quantachrome GmbH & Co. KG mittels Laserbeugung nach ISO 13320 (2009) durchgeführt werden.

Die Erfindung betrifft auch ein Verfahren zur Herstellung des erfindungsgemäßen Aluminiumoxid-Keramikmaterials, bei dem

(a) mindestens ein Aluminiumoxid enthaltendes Ausgangspulver verpresst wird, insbesondere bei einem Druck von 10 bis 1300 MPa und bevorzugt 500 bis 1000 MPa, um einen Pulverpressling zu erhalten, und
(b) gegebenenfalls der Pulverpressling vorgesintert wird, insbesondere bei einer Temperatur von 400 bis 1000°C, bevorzugt 500 bis 800 und besonders bevorzugt 600 bis 700°C, um einen vorgesinterten Keramikkörper zu erhalten,

wobei das Ausgangspulver, der Pulverpressling und/oder der vorgesinterte Keramikkörper mit einer Lösung in Kontakt gebracht werden, die ein Europiumsalz und gegebenenfalls ein Neodymsalz enthält.

In einer bevorzugten Ausführungsform des erfindungsgemäßen Verfahrens wird der Pulverpressling und/oder der vorgesinterte Keramikkörper mit einem Europiumsalz und gegebenenfalls mit einem Neodymsalz infiltriert. Geeignete Infiltrationsverfahren sind beispielsweise in der US 2014/135200 A1 beschrieben.

In einer weiteren bevorzugten Ausführungsform des erfindungsgemäßen Verfahrens wird das Ausgangspulver mit einem Europiumsalz und gegebenenfalls mit einem Neodymsalz beschichtet. Geeignete Beschichtungsverfahren sind beispielsweise in der US 2007/292597 A1 beschrieben.

Besonders bevorzugt werden mindestens zwei, insbesondere mindestens drei und vorzugsweise mindestens vier Aluminiumoxid enthaltende Ausgangspulver verwendet, die sich in ihrer Farbe unterscheiden. Die Ausgangspulver werden in Form von Schichten und vorzugsweise in Form eines Gradienten, der eine kontinuierliche Änderung der Farbe aufweist, aufeinander angeordnet.

Die Erfindung betrifft zudem auch ein Verfahren zur Herstellung eines gesinterten Aluminiumoxid-Keramikkörpers, bei dem das erfindungsgemäße Aluminiumoxid-Keramikmaterial bei einer Sintertemperatur von 1200 bis 1600°C, insbesondere 1300 bis 1550°C und bevorzugt 1350 bis 1500°C, dichtgesintert wird. Bevorzugt erfolgt das Sintern in einer sauerstoffhaltigen Atmosphäre, insbesondere in Luft, und vorzugsweise bei Umgebungsdruck.

In einer bevorzugten Ausführungsform dieses Verfahrens beträgt der Zeitraum für das Aufheizen des Aluminiumoxid-Keramikmaterials von Raumtemperatur auf die Sintertemperatur für das Dichtsintern, das Halten bei der Sintertemperatur und das Abkühlen auf die Endtemperatur nicht mehr als 150 min, insbesondere nicht mehr als 120 min, bevorzugt nicht mehr als 60 min, besonders bevorzugt nicht mehr als 40 min und am meisten bevorzugt nicht mehr als 20 min. Dabei wird unter "Endtemperatur" eine Temperatur verstanden, bei der der gesinterte Aluminiumoxid-Keramikkörper in die Hand genommen werden kann, und sie beträgt insbesondere 15 bis 80°C, bevorzugt 25 bis 60°C und besonders bevorzugt etwa 50°C. Unter "Raumtemperatur" wird eine Temperatur von insbesondere 15 bis 30°C, bevorzugt 20 bis 25°C und besonders bevorzugt etwa 25°C verstanden.

Die Aufheizgeschwindigkeit beträgt insbesondere mehr als 50 K/min, bevorzugt mehr als 100 K/min und besonders bevorzugt mehr als 200 K/min. Die Haltezeit beträgt insbesondere weniger als 30 min, bevorzugt weniger als 20 min und besonders bevorzugt weniger als 10 min. Die Abkühlgeschwindigkeit von der Sintertemperatur auf die Endtemperatur beträgt insbesondere mehr als 50 K/min, bevorzugt mehr als 100 K/min und besonders bevorzugt mehr als 150 K/min.

Die nach der Dichtsinterung erhaltene dentale Restauration kann gegebenenfalls noch mit einer Verblendung versehen, poliert und/oder glasiert werden.

Das erfindungsgemäße Verfahren eignet sich unter anderem für die Herstellung von optischen und technischen Bauteilen, Schmuck und Uhren.

In besonderer Weise eignet sich das erfindungsgemäße Verfahren für die Herstellung von dentalen Restaurationen. In einer bevorzugten Ausführungsform handelt es sich daher bei dem gesinterten Aluminiumoxid-Keramikkörper um eine dentale Restauration und insbesondere um eine Brücke, ein Inlay, ein Onlay, eine Krone, ein Implantat, ein Veneer, eine Schale oder ein Abutment. Ganz besonders eignet sich das erfindungsgemäße Verfahren zur Herstellung von dentalen Restaurationen, insbesondere Brücken, die zwei oder mehr Glieder umfassen. Dabei ist ein Verfahren besonders bevorzugt, bei dem dem Aluminiumoxid-Keramikmaterial vor dem Dichtsintern, insbesondere durch maschinelle Bearbeitung und bevorzugt mittels Schleifen oder Fräsen, die Form der dentalen Restauration gegeben wird.

Zur Herstellung einer dentalen Restauration wird das erfindungsgemäße Aluminiumoxid-Keramikmaterials vorzugsweise in Form eines Rohlings eingesetzt. Der Rohling kann eine beliebige Form haben. Dabei wird insbesondere eine Form gewählt, die eine einfache maschinelle Bearbeitung des Rohlings in üblichen dentalen Schleif- und Fräsvorrichtungen gestattet. Der Rohling liegt bevorzugt als Block, Block mit einer Schnittstelle, wie z.B. einem Loch als Implantatschnittstelle, Scheibe oder zahnähnliche Vorform (Preform) vor. Dabei ist es bevorzugt, dass der Rohling auch eine Haltevorrichtung, wie einen Haltezapfen, aufweist, welche einstückig mit dem Rohling ausgebildet ist. Denn dadurch wird ein bisher übliches späteres Anbringen eines Halters durch Ankleben überflüssig gemacht. Die Haltevorrichtung dient der Aufnahme des Rohlings in einer Bearbeitungsvorrichtung, wie einer CAM-Maschine.

Die Erfindung betrifft auch die Verwendung des erfindungsgemäßen Aluminiumoxid-Keramikmaterials zur Herstellung einer dentalen Restauration und insbesondere einer Brücke, eines Inlays, eines Onlays, einer Krone, eines Implantats, eines Veneers, einer Schale oder eines Abutments.

Bevorzugte Ausführungsformen der Verwendung sind wie oben für das erfindungsgemäße Verfahren beschrieben.

Die Erfindung wird im Folgenden anhand von Beispielen näher erläutert.

**Beispiele**

**Allgemeine Verfahrensweise: Sinterprogramme**

**Standardsinterprogramm (770 min) :**

| Temperatur | Dauer |
|---|---|
| ca. 25°C bis 700°C | 28 min |
| 700°C bis 1350°C | 217 min |
| 1350°C bis 1425°C | 75 min |
| Halten bei 1425°C | 60 min |
| 1425°C bis 350°C | 358 min |
| 350°C bis 300°C | 17 min |
| 300°C bis ca. 50°C | 15 min |

**Schnellsinterprogramm 1 (130 min):**

| Temperatur | Dauer |
|---|---|
| ca. 25°C bis 900°C | 10 min |
| 900°C bis 1470°C | 38 min |
| Halten bei 1470°C | 60 min |
| 1470°C bis 1100°C | 7 min |
| 1100°C bis ca. 50°C | 15 min |

**Schnellsinterprogramm 2 (36 min):**

| Temperatur | Dauer |
|---|---|
| ca. 25°C bis 500°C | 1,25 min |
| 500°C bis 1300°C | 2 min |
| 1300°C bis 1390°C | 0,25 min |
| Halten bei 1390°C | 30 min |
| 1390°C bis 1200°C | 0,5 min |
| 1200°C bis ca. 50°C | 2 min |

**Schnellsinterprogramm 3 (18 min):**

| Temperatur | Dauer |
|---|---|
| ca. 25°C bis 500°C | 1,25 min |
| 500°C bis 1300°C | 2 min |
| 1300°C bis 1470°C | 2 min |

(fortgesetzt)

| Temperatur | Dauer |
|---|---|
| Halten bei 1470°C | 10 min |
| 1470°C bis 1200°C | 0,75 min |
| 1200°C bis ca. 50°C | 2 min |

Das Sintern erfolgte in allen Fällen unter Umgebungsdruck und Luftatmosphäre.

**Beispiel 1**

Aus einem kommerziell erhältlichen Aluminiumoxid-Pulver (TAIMICRON DS-UF-92M, Taimei Chemicals CO., Ltd. Japan), das 99,95 Gew.-% $Al_2O_3$ und 0,05 Gew.-% MgO enthielt, wurden durch uniaxiales Pressen bei einem Druck von 530 MPa und anschließendes Vorsintern bei einer Temperatur von 675°C für eine Dauer von 120 min zylindrische Fräsrohlinge mit einem Durchmesser von 60 mm hergestellt. Aus diesen Fräsrohlingen wurden durch Fräsen mit einer Fräsmaschine vom Typ PrograMill PM7 unter Verwendung von Fräswerkzeugen vom Typ PrograMill tool yellow 1.0 und 2.5 (Ivoclar Vivadent AG, Liechtenstein) scheibenförmige Prüfkörper mit einer Höhe von ca. 1,6 bis 2,0 mm und einem Durchmesser von ca. 15 bis 17 mm gebildet. Die Prüfkörper wurden in 3 Gruppen aufgeteilt.

Die Prüfkörper der Gruppe 1 (Vergleich) wurden nicht mit Europium oder Neodym infiltriert.

Die Prüfkörper der Gruppe 2 (erfindungsgemäß) wurden 10 s lang mit einer wässrigen Lösung infiltriert, die 6 Gew.-% Europiumnitrat enthielt, und anschließend bei 80°C für 2 h in einem Trockenschrank getrocknet. Dadurch wurden Prüfkörper erhalten, die 0,37 Gew.-% Europium, berechnet als $Eu_2O_3$, enthielten.

Die Prüfkörper der Gruppe 3 (erfindungsgemäß) wurden 10 s lang mit einer wässrigen Lösung infiltriert, die 8 Gew.-% Europiumnitrat und 4 Gew.-% Neodymnitrat enthielt, und anschließend bei 80°C für 2 h in einem Trockenschrank getrocknet. Dadurch wurden Prüfkörper erhalten, die 0,54 Gew.-% Europium, berechnet als $Eu_2O_3$, und 0,26 Gew.-% Neodym, berechnet als $Nd_2O_3$, enthielten.

Die Prüfkörper aus allen drei Gruppen wurden mit dem Standardsinterprogramm, dem Schnellsinterprogramm 1, dem Schnellsinterprogramm 2 oder dem Schnellsinterprogramm 3 gemäß der allgemeinen Verfahrensweise dichtgesintert. Danach wurden die Prüfkörper mit einer Poliermaschine vom Typ Buehler AutoMet 250 (Buehler, ITW Test & Measurement GmbH, Esslingen, Germany) mit einem Anpressdruck von 5 N jeweils 12 min mit einer 35 $\mu$m-Diamantschleifscheibe und anschließend 18 min mit einer 15 $\mu$m-Diamantsuspension auf einer Polierscheibe poliert.

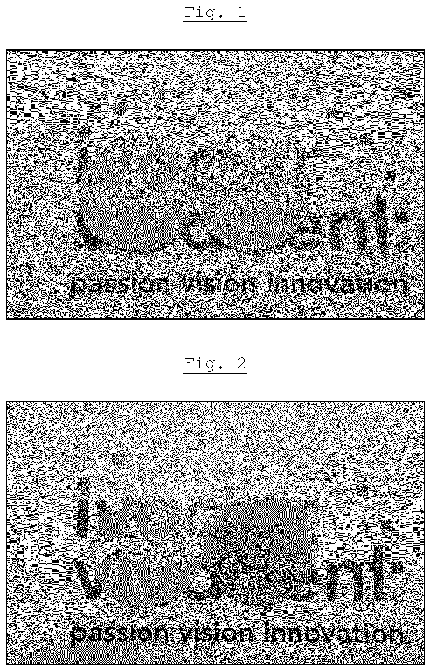

Fig. 1 zeigt einen visuellen Vergleich eines Prüfkörpers der Gruppe 2 (links) und eines Prüfkörpers der Gruppe 1 (rechts) nach dem Standardsinterprogramm (770 min).

Fig. 2 zeigt einen visuellen Vergleich eines Prüfkörpers der Gruppe 2 (links) und eines Prüfkörpers der Gruppe 1 (rechts) nach dem Schnellsinterprogramm 1 (130 min).

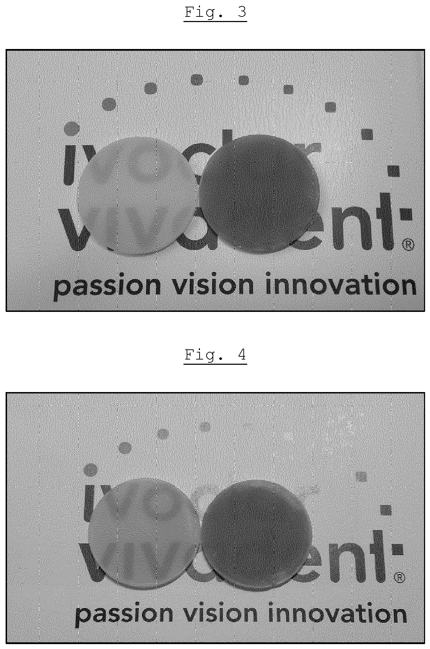

Fig. 3 zeigt einen visuellen Vergleich eines Prüfkörpers der Gruppe 3 (links) und eines Prüfkörpers der Gruppe 1 (rechts) nach dem Schnellsinterprogramm 2 (36 min).

Fig. 4 zeigt einen visuellen Vergleich eines Prüfkörpers der Gruppe 2 (links) und eines Prüfkörpers der Gruppe 1 (rechts) nach dem Schnellsinterprogramm 3 (18 min).

Die Figuren zeigen deutlich, dass bei den erfindungsgemäßen Prüfkörpern der Gruppen 2 und 3 im Vergleich zu den Prüfkörpern der Gruppe 1 eine unerwünschte grau-braune Verfärbung zumindest weitgehend vermieden und zugleich eine deutlich bessere Transluzenz erhalten wird.

An Prüfkörpern, die mit dem Standardsinterprogramm bzw. dem Schnellsinterprogramm 1 dichtgesintert wurden, wurden zudem die biaxiale Biegefestigkeit gemäß DIN EN ISO 6872 und die Risszähigkeit gemäß ISO 14627:2012 gemessen. Die Ergebnisse sind in der nachfolgenden Tabelle wiedergegeben:

| Gruppe | 1 | 2 | 3 |
|---|---|---|---|
| Eu als $Eu_2O_3$ [Gew.-%] | - | 0,37 | 0,54 |
| Nd als $Nd_2O_3$ [Gew.-%] | - | - | 0,26 |
| biaxiale Biegefestigkeit [MPa] nach Standardsinterprogramm | 573 | 555 | 641 |

(fortgesetzt)

| Gruppe | 1 | 2 | 3 |
|---|---|---|---|
| biaxiale Biegefestigkeit [MPa] nach Schnellsinterprogramm 1 | 566 | 617 | |
| Risszähigkeit [MPa·m$^{0,5}$] nach Standardsinterprogramm | 3,47 | 3,49 | |
| Risszähigkeit [MPa·m$^{0,5}$] nach Schnellsinterprogramm 1 | 3,48 | 3,50 | |

**Beispiel 2**

Aus einem kommerziell erhältlichen Aluminiumoxid-Pulver (TAIMICRON DS-UF-92M, Taimei Chemicals CO., Ltd. Japan), das 99,95 Gew.-% $Al_2O_3$ und 0,05 Gew.-% MgO enthielt, wurden durch uniaxiales Pressen bei einem Druck von 950 MPa und anschließendes Vorsintern bei einer Temperatur von 675°C für eine Dauer von 120 min zylindrische Fräsrohlinge mit einem Durchmesser von 60 mm hergestellt. Aus diesen Fräsrohlingen wurden durch Fräsen mit einer Fräsmaschine vom Typ PrograMill PM7 unter Verwendung von Fräswerkzeugen vom Typ PrograMill tool yellow 1.0 und 2.5 (Ivoclar Vivadent AG, Liechtenstein) scheibenförmige Prüfkörper mit einer Höhe von ca. 1,3 bis 1,4 mm und einem Durchmesser von ca. 13 bis 14 mm gebildet.

Die Prüfkörper der Gruppe 1 (Vergleich) wurden nicht mit Europium oder Neodym infiltriert.

Die Prüfkörper der Gruppe 2 (erfindungsgemäß) wurden 10 s lang mit einer wässrigen Lösung infiltriert, die 6 Gew.-% Europiumnitrat enthielt, und anschließend bei 80°C für 2 h in einem Trockenschrank getrocknet. Dadurch wurden Prüfkörper erhalten, die 0,36 Gew.-% Europium, berechnet als $Eu_2O_3$, enthielten.

Die Prüfkörper der Gruppe 3 (erfindungsgemäß) wurden 10 s lang mit einer wässrigen Lösung infiltriert, die 8 Gew.-% Europiumnitrat und 4 Gew.-% Neodymnitrat enthielt, und anschließend bei 80°C für 2 h in einem Trockenschrank getrocknet. Dadurch wurden Prüfkörper erhalten, die 0,54 Gew.-% Europium, berechnet als $Eu_2O_3$, und 0,26 Gew.-% Neodym, berechnet als $Nd_2O_3$, enthielten.

Die Prüfkörper aus allen drei Gruppen wurden mit dem Schnellsinterprogramm 1 gemäß der allgemeinen Verfahrensweise dichtgesintert. Danach wurden die Prüfkörper mit einer Poliermaschine vom Typ Buehler AutoMet 250 (Buehler, ITW Test & Measurement GmbH, Esslingen, Germany) mit einem Anpressdruck von 5 N jeweils 12 min mit einer 35 μm-Diamantschleifscheibe und anschließend 18 min mit einer 15 μm-Diamantsuspension auf einer Polierscheibe poliert. Nach dem Sintern und Polieren wiesen die Proben eine Höhe von ca. 1 mm und einen Durchmesser von ca. 10 mm auf.

Anschließend wurden die Transluzenz und die Farbwerte gemessen. Die Ergebnisse sind in der nachfolgenden Tabelle wiedergegeben:

| Gruppe | 1 | 2 | 3 |
|---|---|---|---|
| Eu als $Eu_2O_3$ [Gew.-%] | - | 0,36 | 0,54 |
| Nd als $Nd_2O_3$ [Gew.-%] | - | - | 0,26 |
| Transluzenz [%] | 29,7 | 52,2 | 44,4 |
| L* | 68,15 | 95,79 | 89,06 |
| * a | 5,2 | -1,58 | -0,83 |
| b* | 14,15 | 6,15 | -1,87 |

Die Ergebnisse entsprechen einem Mittelwert aus jeweils 6 Prüfkörpern und sind auf eine Probendicke von 1,012 mm normiert.

Man erkennt, dass insbesondere die Transluzenzwerte der erfindungsgemäßen Prüfkörper der Gruppen 2 und 3 im Vergleich zu den Prüfkörpern der Gruppe 1 sehr deutlich verbessert sind.

**Beispiel 3**

Aus einem kommerziell erhältlichen Aluminiumoxid-Pulver (TAIMICRON DS-UF-92M, Taimei Chemicals CO., Ltd. Japan), das 99,95 Gew.-% $Al_2O_3$ und 0,05 Gew.-% MgO enthielt, wurden durch uniaxiales Pressen bei einem Druck von 510 MPa und anschließendes Vorsintern bei einer Temperatur von 675°C für eine Dauer von 120 min zylindrische Fräsrohlinge mit einem Durchmesser von 60 mm hergestellt. Aus diesen Fräsrohlingen wurden durch Fräsen mit einer Fräsmaschine vom Typ PrograMill PM7 unter Verwendung von Fräswerkzeugen vom Typ PrograMill tool yellow 1.0 und 2.5 (Ivoclar Vivadent AG, Liechtenstein) scheibenförmige Prüfkörper mit einer Höhe von ca. 1,3 bis 1,4 mm und einem Durchmesser von ca. 13 bis 14 mm gebildet.

Die Prüfkörper der Gruppe 1 (Vergleich) wurden nicht mit Europium oder Neodym infiltriert.

Die Prüfkörper der Gruppe 2 (erfindungsgemäß) wurden 10 s lang mit einer wässrigen Lösung infiltriert, die 6 Gew.-% Europiumnitrat enthielt, und anschließend bei 80°C für 2 h in einem Trockenschrank getrocknet. Dadurch wurden Prüfkörper erhalten, die 0,36 Gew.-% Europium, berechnet als $Eu_2O_3$, enthielten.

Die Prüfkörper der Gruppe 3 (erfindungsgemäß) wurden 10 s lang mit einer wässrigen Lösung infiltriert, die 8 Gew.-% Europiumnitrat und 4 Gew.-% Neodymnitrat enthielt, und anschließend bei 80°C für 2 h in einem Trockenschrank getrocknet. Dadurch wurden Prüfkörper erhalten, die 0,54 Gew.-% Europium, berechnet als $Eu_2O_3$, und 0,26 Gew.-% Neodym, berechnet als $Nd_2O_3$, enthielten.

Die Prüfkörper aus allen drei Gruppen wurden mit dem Schnellsinterprogramm 1 gemäß der allgemeinen Verfahrensweise dichtgesintert. Danach wurden die Prüfkörper mit einer Poliermaschine vom Typ Buehler AutoMet 250 (Buehler, ITW Test & Measurement GmbH, Esslingen, Germany) mit einem Anpressdruck von 5 N jeweils 12 min mit einer 35 μm-Diamantschleifscheibe und anschließend 18 min mit einer 15 μm-Diamantsuspension auf einer Polier-scheibe poliert. Nach dem Sintern und Polieren wiesen die Proben eine Höhe von ca. 1 mm und einen Durchmesser von ca. 10 mm auf.

Anschließend wurde die Korngröße gemessen. Die Ergebnisse sind in der nachfolgenden Tabelle wiedergegeben:

| Gruppe | 1 | 2 | 3 |
|---|---|---|---|
| Eu als $Eu_2O_3$ [Gew.-%] | - | 0,36 | 0,54 |
| Nd als $Nd_2O_3$ [Gew.-%] | - | - | 0,26 |
| Korngröße [μm] | 1,657 ± 0,207 | 1,271 ± 0,207 | 1,430 ± 0,239 |

Außerdem wurden REM-Analysen der Probenkörper vorgenommen.

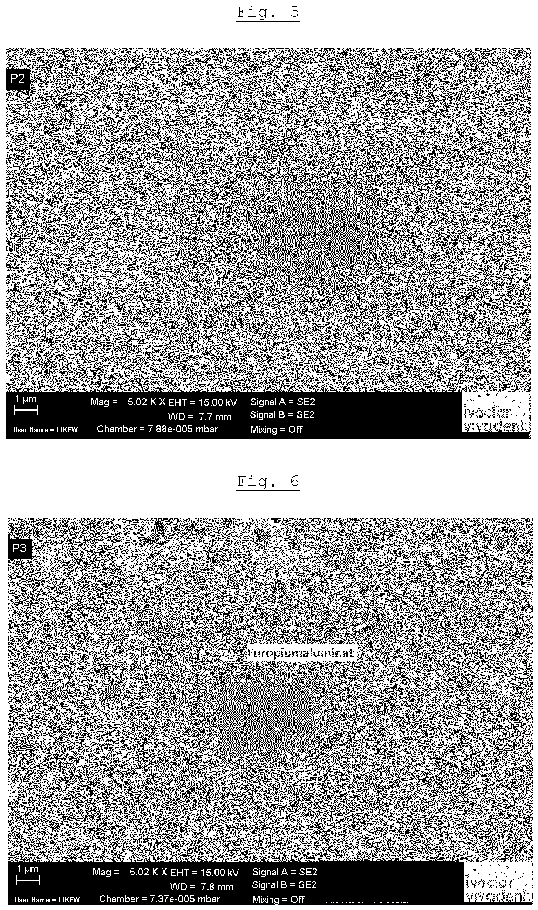

Fig. 5 zeigt eine REM-Aufnahme eines Probenkörpers der Gruppe 1.

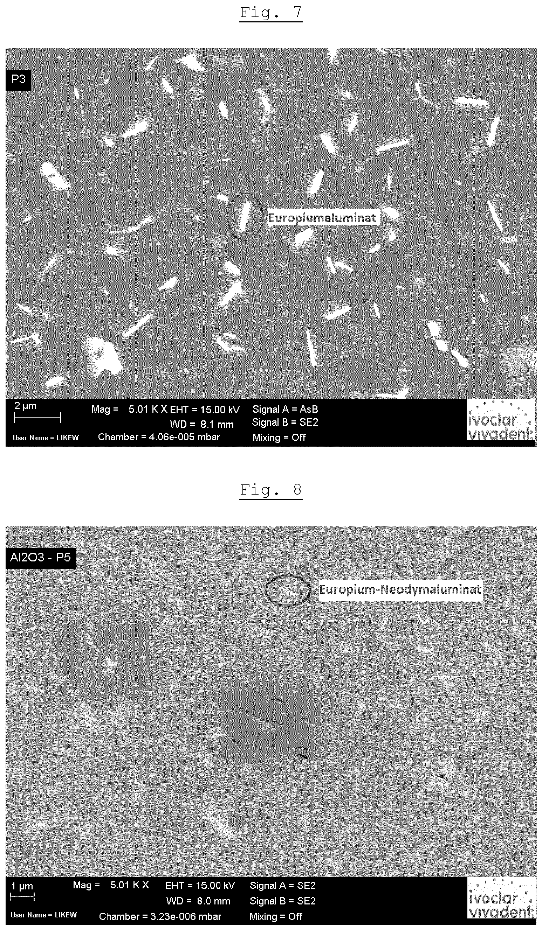

Fig. 6 und 7 zeigen REM-Aufnahmen von Probenkörpern der Gruppe 2. Darin erkennt man deutlich stäbchenförmige und plättchenförmige Europiumaluminat-Kristalle, die aufgrund der hohen Ordnungszahl von Europium im REM eine hohe Helligkeit haben.

Fig. 8 zeigt eine REM-Aufnahme eines Probenkörpers der Gruppe 3. Darin erkennt man deutlich stäbchenförmige und plättchenförmige Europium-Neodymaluminat-Kristalle, die aufgrund der hohen Ordnungszahlen von Europium und Neodym im REM ebenfalls eine hohe Helligkeit haben.

**Patentansprüche**

1.  Aluminiumoxid-Keramikmaterial, das die folgenden Komponenten enthält:

| Komponente | Gew.-% |
|---|---|
| $Al_2O_3$ | 95,0 bis 99,989 |
| MgO | 0,001 bis 0,1 |
| Eu, berechnet als $Eu_2O_3$ | 0,01 bis 1,0. |

2.  Aluminiumoxid-Keramikmaterial nach Anspruch 1, das mindestens 96,4, insbesondere mindestens 97,4 und bevorzugt mindestens 98,4 Gew.-% $Al_2O_3$ enthält und vorzugsweise 96,4 bis 99,9, insbesondere 97,4 bis 99,85, bevorzugt 98,4 bis 99,8, weiter bevorzugt 99,4 bis 99,75, besonders bevorzugt 99,5 bis 99,7 und am meisten bevorzugt 99,55 bis 99,65 Gew.-% $Al_2O_3$ enthält.

3.  Aluminiumoxid-Keramikmaterial nach Anspruch 1 oder 2, das 0,005 bis 0,09, insbesondere 0,01 bis 0,08, bevorzugt 0,03 bis 0,07 und besonders bevorzugt 0,04 bis 0,06 Gew.-% MgO enthält.

4.  Aluminiumoxid-Keramikmaterial nach einem der Ansprüche 1 bis 3, das 0,05 bis 0,9, insbesondere 0,1 bis 0,8, bevorzugt 0,2 bis 0,7 und besonders bevorzugt 0,3 bis 0,6 Gew.-% Eu, berechnet als $Eu_2O_3$, enthält.

5. Aluminiumoxid-Keramikmaterial nach einem der Ansprüche 1 bis 4, das 0,01 bis 1,0, insbesondere 0,05 bis 0,5, bevorzugt 0,1 bis 0,4 und besonders bevorzugt 0,2 bis 0,3 Gew.-% Nd, berechnet als $Nd_2O_3$, enthält.

6. Aluminiumoxid-Keramikmaterial nach einem der Ansprüche 1 bis 5, das 0,001 bis 2,0, insbesondere 0,01 bis 1,0, bevorzugt 0,05 bis 0,8 und besonders bevorzugt 0,1 bis 0,5 Gew.-% färbende Oxide enthält, wobei die färbenden Oxide vorzugsweise ausgewählt sind aus Oxiden von Fe, Cr, Mn, Co, Cu, Ag, Er, Pr, Tb, Ce, Ho, Zr und La.

7. Aluminiumoxid-Keramikmaterial nach einem der Ansprüche 1 bis 7, das mindestens zwei, insbesondere mindestens drei und vorzugsweise mindestens vier Schichten, die sich in ihrer Farbe unterscheiden, und vorzugsweise einen Gradienten umfasst, der eine kontinuierliche Änderung der Farbe aufweist.

8. Aluminiumoxid-Keramikmaterial nach einem der Ansprüche 1 bis 7, das nicht mehr als 0,5, insbesondere nicht mehr als 0,3, bevorzugt nicht mehr als 0,1, besonders bevorzugt nicht mehr als 0,05 und am meisten bevorzugt nicht mehr als 0,01 Gew.-% andere Oxide enthält.

9. Aluminiumoxid-Keramikmaterial nach einem der Ansprüche 1 bis 8, das eine Europiumaluminat-Kristallphase enthält, die vorzugsweise stäbchenförmige oder plättchenförmige Europiumaluminat-Kristalle enthält.

10. Aluminiumoxid-Keramikmaterial nach Anspruch 9, das stäbchenförmige Europiumaluminat-Kristalle enthält, welche bevorzugt eine mittlere Breite von 0,01 bis 0,25, insbesondere von 0,05 bis 0,20, bevorzugt 0,10 bis 0,15 und besonders bevorzugt 0,11 bis 0,13 $\mu$m haben und/oder ein mittleres Verhältnis von Breite zu Länge von 1:1 bis 1:10, insbesondere 1:2 bis 1:9, bevorzugt 1:4 bis 1:8 und besonders bevorzugt 1:6 bis 1:7 aufweisen.

11. Aluminiumoxid-Keramikmaterial nach einem der Ansprüche 1 bis 10, das nicht dichtgesintert und insbesondere vorgesintert ist.

12. Aluminiumoxid-Keramikmaterial nach einem der Ansprüche 1 bis 11, das eine relative Dichte im Bereich von 45 bis 90%, insbesondere im Bereich von 50 bis 80% und bevorzugt im Bereich von 55 bis 70%, jeweils bezogen auf die Reindichte des Aluminiumoxid-Keramikmaterials, aufweist.

13. Verfahren zur Herstellung des Aluminiumoxid-Keramikmaterials nach einem der Ansprüche 1 bis 12, bei dem

(a) mindestens ein Aluminiumoxid enthaltendes Ausgangspulver verpresst wird, insbesondere bei einem Druck von 10 bis 1300 MPa und bevorzugt 500 bis 1000 MPa, um einen Pulverpressling zu erhalten, und
(b) gegebenenfalls der Pulverpressling vorgesintert wird, insbesondere bei einer Temperatur von 400 bis 1000°C, bevorzugt 500 bis 800 und besonders bevorzugt 600 bis 700°C, um einen vorgesinterten Keramikkörper zu erhalten,

wobei das Ausgangspulver, der Pulverpressling und/oder der vorgesinterte Keramikkörper mit einer Lösung in Kontakt gebracht werden, die ein Europiumsalz und gegebenenfalls ein Neodymsalz enthält.

14. Verfahren nach Anspruch 13, bei dem mindestens zwei, insbesondere mindestens drei und vorzugsweise mindestens vier Aluminiumoxid enthaltende Ausgangspulver verwendet werden, die sich in ihrer Farbe unterscheiden, wobei die Ausgangspulver in Form von Schichten und vorzugsweise in Form eines Gradienten, der eine kontinuierliche Änderung der Farbe aufweist, aufeinander angeordnet werden.

15. Verfahren zur Herstellung eines gesinterten Aluminiumoxid-Keramikkörpers, bei dem das Aluminiumoxid-Keramikmaterial nach einem der Ansprüche 1 bis 12 bei einer Sintertemperatur von 1200 bis 1600°C, insbesondere 1300 bis 1550°C und bevorzugt 1350 bis 1500°C, dichtgesintert wird.

16. Verfahren nach Anspruch 15, bei dem der Zeitraum für das Aufheizen des Aluminiumoxid- Keramikmaterials von Raumtemperatur auf die Sintertemperatur, das Halten bei der Sintertemperatur und das Abkühlen auf die Endtemperatur nicht mehr als 150 min, insbesondere nicht mehr als 120 min, bevorzugt nicht mehr als 60 min, besonders bevorzugt nicht mehr als 40 min und am meisten bevorzugt nicht mehr als 20 min beträgt.

17. Verfahren nach Anspruch 15 oder 16, bei dem der gesinterte Aluminiumoxid-Keramikkörper eine Transluzenz im Bereich von 35 bis 100%, insbesondere im Bereich von 40 bis 99%, bevorzugt im Bereich von 45 bis 98% und besonders bevorzugt im Bereich von 50 bis 97%, aufweist.

18. Verfahren nach einem der Ansprüche 15 bis 17, bei dem der gesinterte Aluminiumoxid-Keramikkörper eine dentale Restauration und insbesondere eine Brücke, ein Inlay, ein Onlay, eine Krone, ein Implantat, ein Veneer, eine Schale oder ein Abutment ist.

19. Verfahren nach Anspruch 18, bei dem dem Aluminiumoxid-Keramikmaterial vor dem Dichtsintern, insbesondere durch maschinelle Bearbeitung und bevorzugt mittels Schleifen oder Fräsen, die Form der dentalen Restauration gegeben wird.

20. Verwendung des Aluminiumoxid-Keramikmaterials nach einem der Ansprüche 1 bis 12 zur Herstellung einer dentalen Restauration und insbesondere einer Brücke, eines Inlays, eines Onlays, einer Krone, eines Implantats, eines Veneers, einer Schale oder eines Abutments.

Fig. 1

Fig. 2

Fig. 3

Fig. 4

Fig. 5

Fig. 6

Fig. 7

Fig. 8

Europäisches
Patentamt

European
Patent Office

Office européen
des brevets

# EUROPÄISCHER RECHERCHENBERICHT

Nummer der Anmeldung

EP 21 19 1484

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 106 242 537 A (SHANGHAI YURONG NEW MAT TECH CO LTD) 21. Dezember 2016 (2016-12-21) * Ansprüche 5-8; Abbildung 1; Beispiele 2,3; Tabelle 1 * ----- | 1-4,6, 8-10, 15-17 | INV. C04B35/115 A61C13/083 A61K6/807 A61K6/822 |
| X | KANG ZHANG ET AL: "Effect of MgO/Eu2O3 Co-doping on the microwave dielectric properties of Al2O3 ceramics", JOURNAL OF INORGANIC MATERIALS – WUJI CAILIAO XUEBAO, Bd. 30, Nr. 9, 2015, Seiten 984-988, XP055842155, CN ISSN: 1000-324X, DOI: 10.15541/jim20150081 * 1. "Experimental method"; Abbildungen 1-3 * ----- | 1-4, 8-10,15, 17 | |
| Y | EP 2 025 659 A1 (3M INNOVATIVE PROPERTIES CO [US]) 18. Februar 2009 (2009-02-18) * Seite 13, Absätze 7-11,14,24,25,27-29,104-106; Ansprüche 1-4,11-14 * ----- | 1-20 | |
| Y | EP 2 098 495 A2 (SEIKO EPSON CORP [JP]) 9. September 2009 (2009-09-09) * Absätze [0196], [0197]; Beispiele 1-4; Tabelle 1 * ----- -/-- | 1-20 | |

RECHERCHIERTE SACHGEBIETE (IPC)

C04B
A61C
A61K

Der vorliegende Recherchenbericht wurde für alle Patentansprüche erstellt

| Recherchenort | Abschlußdatum der Recherche | Prüfer |
|---|---|---|
| München | 10. Dezember 2021 | Bonneau, Sébastien |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

Seite 1 von 2

Europäisches
Patentamt

European
Patent Office

Office européen
des brevets

## EUROPÄISCHER RECHERCHENBERICHT

Nummer der Anmeldung

EP 21 19 1484

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | LIU JUNCHANG ET AL: "Effects of Eu2O3 addition on microstructure, grain-boundary cohesion and wear resistance of high-alumina ceramics", JOURNAL OF ALLOYS AND COMPOUNDS, Bd. 695, Februar 2017 (2017-02), Seiten 2324-2329, XP055868751, CH ISSN: 0925-8388, DOI: 10.1016/j.jallcom.2016.11.099 * das ganze Dokument * ----- | 1-20 | |
| | | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 10. Dezember 2021 | Bonneau, Sébastien |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes
Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 19 1484

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-12-2021

| Im Recherchenbericht angeführtes Patentdokument | Datum der Veröffentlichung | Mitglied(er) der Patentfamilie | Datum der Veröffentlichung |
|---|---|---|---|
| CN 106242537 A | 21-12-2016 | KEINE | |
| EP 2025659 A1 | 18-02-2009 | AT 524425 T | 15-09-2011 |
| | | CN 101778807 A | 14-07-2010 |
| | | EP 2025659 A1 | 18-02-2009 |
| | | EP 2176194 A2 | 21-04-2010 |
| | | JP 2010534245 A | 04-11-2010 |
| | | US 2010221683 A1 | 02-09-2010 |
| | | WO 2009014903 A2 | 29-01-2009 |
| EP 2098495 A2 | 09-09-2009 | EP 2098495 A2 | 09-09-2009 |
| | | JP 4770851 B2 | 14-09-2011 |
| | | JP 2009209019 A | 17-09-2009 |
| | | US 2009224442 A1 | 10-09-2009 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007065914 A1
- EP 2098188 A1
- EP 2101133 A1
- WO 2012057829 A2
- WO 2015091744 A1
- WO 2015121364 A1
- US 2014135200 A1
- US 2007292597 A1

**In der Beschreibung aufgeführte Nicht-Patentliteratur**

- **M. I. MENDELSON.** *J. Am. Ceram. Soc.,* 1969, vol. 52 (8), 443-446